# DEMANDE DE BREVET EUROPEEN

(11) **EP 1 902 721 A1**
(43) Date de publication de la demande: **26.03.2008**
(21) Numéro de dépôt: 06291475.9
(22) Date de dépôt: 20.09.2006
(51) Int. Cl.: A61K 36/45, A61P 31/04, A61P 13/02, A61K 31/496, A61K 31/495, A61K 31/535, A61K 31/4178, A61K 31/43

(54) **Compositions médicales et nutritionnelles à base de vaccinium macrocarpon**

(71) Demandeur: Renard, Loïc, 92150 Suresnes (FR); Pharmatoka SAS, 92500 Rueil Malmaison (FR); Haesearts, Gunther, 1970 Wezembeek (BE); Botto, Henry, 75003 Paris (FR)
(72) Inventeur: Renard, Loïc, 92500 Rueil Malmaison (FR)
(74) Mandataire: Burtin, Jean-François

(57) **Abrégé**

L'invention concerne une association d'extrait de Vaccinium macrocarpon (Cranberry) et d'un agent antibactérien urinaire actif contre les bactéries à Gram+ et à Gram-. L'utilisation conjointe d'un agent antimicrobien éliminé par voie rénale, complète et renforce l'action anti-adhérente de l'extrait.
L'association se présente sous forme liquide ou solide après dispersion dans un excipient ou un véhicule inerte non toxique pharmaceutiquement acceptable. Elle peut être utilisée pour le traitement des infections urinaires aiguës ou récidivantes.

## Description

La présente invention se rapporte au domaine de la chimie et plus particulièrement à celui des produits bénéfiques pour la santé.

L'invention concerne plus particulièrement de nouvelles compositions antibactériennes destinées au traitement des infections bactériennes urinaires aiguës ou chroniques.

Les infections urinaires sont d'une extrême fréquence. Elles viennent, après les infections respiratoires, au second rang des motifs de consultation et de prescription d'antibiotiques. Elles sont facilement récidivantes notamment chez la femme et les porteurs de vessie neurologique. L'agent microbien le plus souvent responsable de ces infections urinaires est *Escherichia coli,* bactérie commensale du tube digestif qui peut devenir pathogène par acquisition d'îlots de pathogénicité. Une autre bactérie fréquemment isolée chez les femmes jeunes non ménopausées est le staphylocoque, en particulier *S. saprophyticus* qui peut être à l'origine de pyurie. D'autres agents microbiens sont également responsables d'infections urinaires : *Proteus, Klebsiella, Enterobacter* parmi les Gram-, *Entérocoques* ou *Staphylocoques* parmi les Gram+ ainsi que des levures.

Ces infections bactériennes peuvent devenir persistantes voire même chroniques et il importe de mettre au point des médicaments efficaces mais en même temps sélectifs pour ne pas faire disparaître la flore bactérienne commensale normalement présente dans l'urètre.

Cette problématique a été résolue par l'association antibactérienne qui concerne l'objet de la présente invention. Celle-ci consiste en une association d'extrait de Vaccinium macrocarpon (CRANBERRY) et d'un agent antibactérien urinaire actif contre les bactéries à Gram + et à Gram -. Les propriétés anti-adhérentes de l'extrait de Vaccinium macrocarpon sont bien connues et ont été mises à profit dans de nombreuses infections urinaires pour combattre le développement de la bactériurie. Par ailleurs, les demandeurs ont démontré, par des expériences *in vitro* (cellules urothéliales T24) et in vivo (essais sur *Caenorhabditis elegans*), que l'extrait de Cranberry avait une activité anti-adhérente significativement plus importante comparée à un placebo vis à vis de souches d'*E. coli* uropathogènes.

L'utilisation conjointe d'un agent antibactérien éliminé par voie rénale, complète et renforce nettement l'action anti-adhérente de cet extrait.

Comme agent antibactérien éliminé par voie rénale, on peut citer en particulier les dérivés du furfural comme la furadoïne, le métronidazole, la nitrofurantoïne..., des dérivés de sulfamides comme la sulfadiazine, la sulfamérazine, des dérivés d'antibiotiques du groupe des aminosides (gentamicine, dibekacine, amikacine), la fosfomycine et ses sels, des dérivés de tétracycline (doxycycline, minocycline, auréomycine...) et surtout des agents antibactériens appartenant à la famille des quinolones, fluorées ou non fluorées telles que : l'acide nalidixique, acide oxolinique, la cinnoxacine, la ciprofloxacine, la miloxacine, la norfloxacine, l'ofloxacine, la lévofloxacine, la rufloxacine, la lomefloxacine, la sparfloxacine) ainsi que les antibiotiques du type beta lactamines (pénicillines semi-synthétiques comme l'amoxicilline ou l'ampicilline ou encore des céphalosporines comme la cefoperazone, la ceftazidine ou le cefuroxime.

Le jus de Cranberry ou Grande Airelle est tiré d'un fruit cultivé au Canada et dans le Nord des Etats-Unis. Ce jus de couleur rouge sombre et de saveur astringente est riche en polyphénols et notamment en proanthocyanidines sous forme libre glycosylée (galactoside, rhamnoside, glucoside) ou estérifiée (gallate) comme par exemple la prodelphinidine, la propetunidine, la promalvidine ou la procyanidine. Ces composés existent en général sous forme polymérisée (dimères à hexamères) et possèdent un enchaînement de type A ou de type B selon les deux schémas suivants, la forme trimère étant actuellement préférée : 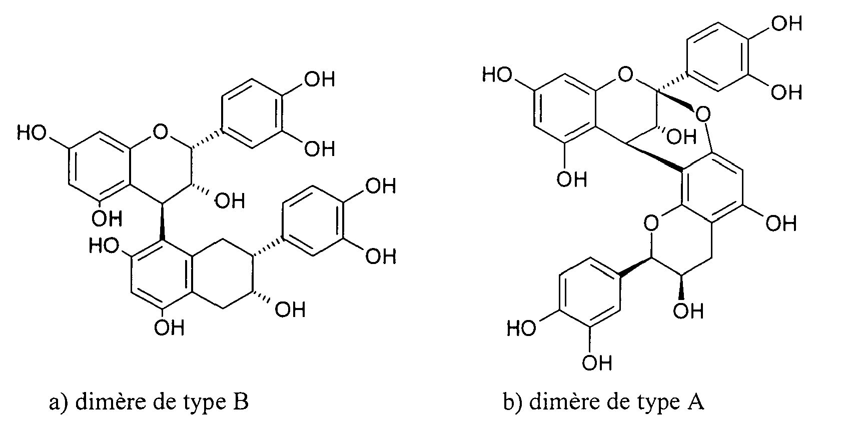 Structure d'un dimère de type B (a) comparé à un dimère (b) de type A

On entend par extrait de Vaccinium macrocarpon, un ensemble de préparations dérivées du jus de Cranberries :
1. le jus de Cranberry naturel contenant de 0,14 à 0,45 % de proanthocyanidines
2. le concentré de jus pur de Cranberry contenant de 1,35 % à 2,50 % de proanthocyanidine
3. les concentrés de jus de Cranberry dont la teneur en polyphénols et notamment en proanthocyanidines obtenus par atomisation sous forme d'une poudre, contiennent de 2,2 % à 4,5 % de proanthocyanidines
4. la poudre concentrée renfermant de 30 à 86 % de polyphénols obtenue par évaporation, concentration, atomisation, puis freeze drying, ou absorption sélective des concentrés sur un matériau poreux ou sur résine échangeuse d'ions.

On inclut dans cette définition les mélanges des différentes préparations susmentionnées en des proportions variables définis par leur teneur en polyphénols totaux et extraits de la Cranberry.

Une forme particulièrement appropriée est un adsorbat de jus de Grande Airelle absorbée sur silice colloïdale, sur polyvinylpyrrolidone réticulée, sur Kaolin ou sur hydroxyde de magnésium.

L'association selon l'invention se présente sous une forme appropriée à l'usage thérapeutique, après dilution par un excipient ou un vecteur inerte, non toxique, pharmaceutiquement acceptable. On pourra citer comme excipient inerte le lactose, la cellulose, le carbonate de calcium, le phosphate tricalcique, le phosphate de magnésium, le stéarate de calcium, le stéarate de magnésium, le talc ou la silice colloïdale (Aerosil (R) 100 ou Aerosil (R) 200).

Comme vecteurs, on pourra citer encore des composés qui favorisent l'excrétion urinaire comme par exemple l'extrait de Fumeterre ou l'extrait d'Orthosiphon.

Les compositions selon l'invention se présentent sous forme solide ou sous forme liquide. Comme formes solides, on pourra citer les comprimés nus ou pelliculés, les dragées, les gélules, les capsules, les pilules, les cachets ou les comprimés orodispersibles. Comme formes liquides, on pourra citer les émulsions, les dispersions ou les suspensions dans un véhicule aqueux ou huileux.

Les compositions selon l'invention sont destinées principalement à la voie orale ou topique. Elles peuvent être utilisées pour la voie parentérale.

Selon la présente invention, les formes solides contiennent de 0,100 à 0,500g d'extrait de jus de Cranberry contenant de 18 à 180 mg de proanthocyanidines de type A et en particulier la fraction utile en terme d'action anti-adhésion bactérienne, allant des dimères aux hexamères -ainsi que 0,100 g à 0,500g d'agent anti-bactérien. Une composition préférée contient de 0,100 à 0,250 de norfloxacine ou de ciprofloxacine par prise unitaire. Selon les besoins, l'agent antimicrobien sera sous forme insoluble dans l'eau (acide oxolinique, nitrofurantoïne, ciprofloxacine, norfloxacine) ou sous la forme d'un sel soluble dans l'eau (sel d'arginine en particulier). Les formes liquides contiennent de préférence de 0,200 à 0,400g d'extrait de Cranberry par ml et de 0,010 g à 0,050 g d'agent anti-bactérien par ml.

L'administration se fait à raison de 1 à 4 prises par jour en fonction de la sévérité et de l'ancienneté de l'infection. Une administration à raison de deux prises par jour (une prise le matin et une prise le soir) est celle qui paraît la plus appropriée pour obtenir un résultat thérapeutique rapide. L'administration peut être poursuivie jusqu'à disparition des signes cliniques de l'infection et jusqu'à élimination de l'agent pathogène dans les urines.

### Exemple 1

### Gélules d'extrait pulvérulent de Grande Airelle et de Ciprofloxacine

■ Extrait pulvérulent de Grande Airelle à 50 % de proanthocyanidines 0,150 kg
■ Ciprofloxacine 0,250 kg
■ Lactose 0,100 kg
■ Talc 0,050 kg
■ Stéarate de magnésium 0,150 kg
pour 1000 gélules

### Exemple II

### Gélules d'extrait de Grande Airelle et de nitrofurantoïne

■ Extrait pulvérulent de Grande Airelle à 50 % de proanthocyanidines 0,200 kg
■ Nitrofurantoïne 0,100 kg
■ Cellulose microcristalline 0,050 kg
■ Stéarate de calcium 0,050 kg
pour 1000 gélules

### Exemple III

### Gélules molles d'extrait de Grande Airelle et d'ofloxacine

■ Extrait de Grande Airelle à 10 % de proanthocyanidines absorbé sur silice colloïdale (Aerosil^{(R)} 200) 0,400 kg
■ Ofloxacine 0,200 kg
■ Poloxamer 188 0,020 kg
■ Huile d'olive 0,100 kg
pour 1000 capsules molles de 0,700 g

### Exemple IV

### Comprimés d'extrait de Grande Airelle et d'Amoxicilline

■ Extrait pulvérulent de Grande Airelle à 25 % 0,200 kg de proanthocyanidines
■ Amoxicilline 0,250 kg
■ Lactose 0,100 kg
■ Mannitol 0,100 kg
■ Talc 0,002 kg
■ Acide stéarique 0,005 kg
pour 1000 comprimés

### Exemple V

### Etude « In vitro » de l'extrait de Vaccinium macrocarpon

L'efficacité de l'extrait de Vaccinium macrocarpon a été évaluée par rapport à un placebo à l'aide d'une étude sur l'activité anti-adhérente bactérienne en utilisant un modèle *in vitro* appliqué sur des urines de volontaires sains.

Cette étude a été menée en double aveugle, randomisée et croisée destinée à comparer les effets de l'administration d'un extrait total de polyphénols extrait de Cranberry par rapport à un placebo chez huit volontaires. Chaque volontaire a reçu en plus de son alimentation normale trois gélules contenant soit un placebo, soit un extrait polyphénolique de Cranberry ou bien encore un régime mixte formé de 2 gélules de placebo et d'une gélule d'extrait de Cranberry. Chaque volontaire a reçu quatre régimes choisis parmi les trois possibilités énoncées ci-dessus avec une période de repos entre chaque régime. Après prise des gélules la veille au soir, les urines du matin ont été recueillies. Quatre souches *d'Escherichia coli* (2 fim H⁺ - pap GII + 1 fim H⁻ pap GII + et 1 fim H - pap GII -) isolées de patients ayant eu une infection urinaire, ont été mises en culture dans les urines des différents volontaires et ont été testées pour évaluer leur capacité à adhérer *in vitro* à des lignées cellulaires urothéliales pour la mise en évidence d'un indice d'adhérence (IA) qui représente le nombre moyen de bactéries par cellule pour 100 cellules. Trois expériences indépendantes ont été réalisées pour chaque essai.

On observe une diminution significative de l'adhérence bactérienne en fonction de la dose absorbée d'extrait de Cranberry. On constate « in vitro » que pour les souches fim H⁺ pap GII⁺ lors de l'administration de 3 gélules d'extrait de Cranberry, l'indice d'adhérence était de 5,18 ± 4,32 alors que par administration de placebo l'IA était de 22,43 ± 3,73 et que lors de l'administration d'une seule gélule d'extrait de Cranberry, l'IA était de 3,37 ± 0,97 (P < 0,001). Pour les souches fim H - pap GII⁺ en présence de 3 gélules d'extrait de Cranberry, l'IA était de 2,78 ± 1,12 alors qu'en présence de placebo, l'IA était de 7,50 ± 1,60 et en présence d'une gélule d'extrait de Cranberry, l'IA était de 4,73 ± 0,87 (P < 0,001).

### Exemple VI

### Méthode « In vitro » sur nématode

L'activité de l'extrait de Vaccinium macrocarpon a été testé *in vivo* sur un modèle utilisant un nématode (lombric). En présence de placebo ou d'une souche d'*E*. *coli* de forte virulence, on constate le décès des vers en 7 jours. En présence d'extrait polyphénolique de Vaccinium macrocarpon, la souche d'*E*. *coli* de forte virulence est moins efficace et n'entraîne le décès d'*E. coli* au bout de 10 jours -sur une souche de faible virulence, l'extrait polyphénolique de Vaccinium macrocarpon n'a pas d'effet remarquable. L'extrait polyphénolique de Vaccinium macrocarpon ait sur *Escherichia coli* en augmentant de 30 % l'espérance de vie des Nématodes.

On peut donc considérer que l'extrait polyphénolique de Vaccinium macrocarpon présente une action anti-adhérente significativement plus importante par rapport au placebo vis-à-vis de souches d'*E. coli* uropathogènes quelque soit la capacité d'adhésion de ces bactéries. Les études « in vitro » et « in vivo » confirment les données cliniques sur les propriétés anti-adhérentes des extraits de Cranberry et démontrent que l'extrait de Cranberry constitue une alternative dans la prévention des infections urinaires.

Le graphique 1 ci-après annexé met en évidence l'effet protecteur vis-à-vis de la létalité des nématodes sur des souches d' *E. coli* uropathogènes de virulence variable.

### Exemple VII

### Etude « in vitro » et « in vivo » de l'adhérence d'une souche uropathogène d'E. coli après administration d'extrait polyphénolique de Cranberry

Les composés polyphénoliques de l'extrait de Cranberry sont capables de modifier la morphologie des souches d' *E. coli,* de dénaturer les adhésines et d'entraîner une modification du passage transmembranaire dans la bactérie.

Il apparaît que les constituants de l'extrait de Cranberry ont la propriété de modifier les souches d'*E. coli* d'une façon qui les rendent incapables de provoquer une infection. Les composés polyphénoliques de cet extrait et en particulier les proanthocyanidines ont une triple action destructrice sur les souches d'*E. coli* :
- elles modifient la morphologie des corps bactériens
- elles changent la nature de la membrane cellulaire
- elles rendent difficile le contact entre les corps bactériens et les cellules par modification des microfibrilles (fimbriac) sur la surface des bactéries *E. coli.* De ce fait, la plus faible adhésion des bactéries diminue fortement leur capacité à mettre en route des phénomènes d'infection.
Le tableau 1 ci-après montre l'effet de l'extrait de Cranberry sur l'adhérence des *E. coli* et sur sa létalité.

**Diminution de la virulence d'E. coli après administration de compostions à base d'extrait de Cranberry**

| Expériences in vitro avec screeming par HBRC et activité anti-adhérence sur des cellules T24 sur 8 volontaires. | | | | | | | |
|---|---|---|---|---|---|---|---|
| **A** | **Titre HBRC et indices d'adhérence ±SD** | | | **Diminution de l'adhérence bactérienne (%)** | | **P** | |
| | **3 PI** | **1UE + 2PI** | **3UE** | **1UE + 2PI** | **3UE** | 3PI vs 3UE | 3PI vs 1UE |
| NECS21963 | 1/128 | 1/8 | 0 | - | - | | - |
| | 21,88 ± 4,42 | 14,22 ± 4,78 | 5,14 ± 6,56 | 35.0 | 76.5 | <0.001 | <0.001 |
| NECS29784 | 1/256 | 1/8 | 0 | - | - | | - |
| | 22,43 ± 3,73 | 14,75 ± 3,45 | 4,38 ± 4,32 | 34.2 | 80.5 | <0.001 | <0.001 |
| NECS | 1/8 | Pur | 0 | - | - | | - |
| | 4,80 ± 0,70 | 3,37 ± 0,97 | 1,74 ± 0,75 | 29.8 | 63.8 | <0.01 | 0.002 |
| NBC13 | NA | NA | NA | - | - | | - |
| | 7,50 ± 1,60 | 4,73 ± 0,87 | 2,78 ± 1,12 | 36.9 | 62.9 | <0.001 | 0.001 |
| | | | | | | | |

| **B** | **DL50 ± SD** | | | **Létalité ± SD** | | **P** | |
|---|---|---|---|---|---|---|---|
| | **3 PI** | | **3 UE** | ***3* PI** | **3 UE** | | |
| NECS21963 | 3.21 ± 0.36 | | 5.43 ± 0.22 | 7 ± 0.25 | 9.5 ± 0.25 | <0.001 | |
| NECS29784 | 3.03 ± 0.22 | | 5.11 ± 0.40 | 6.5 ± 0.25 | 9 ± 0.25 | <0.001 | |
| NEC5 | 5.33 ± 0.15 | | 6.21 ± 0.17 | 11 ± 0.25 | 11.5 ± 0.25 | - | |
| NEC13 | 4.76 ± 0.35 | | 5.28 ± 1.24 | 9 ± 0.25 | 10 ± 0.25 | 0.02 | |

| | | | | | | | |
|---|---|---|---|---|---|---|---|
| NA = sans effet | | | | | | | |

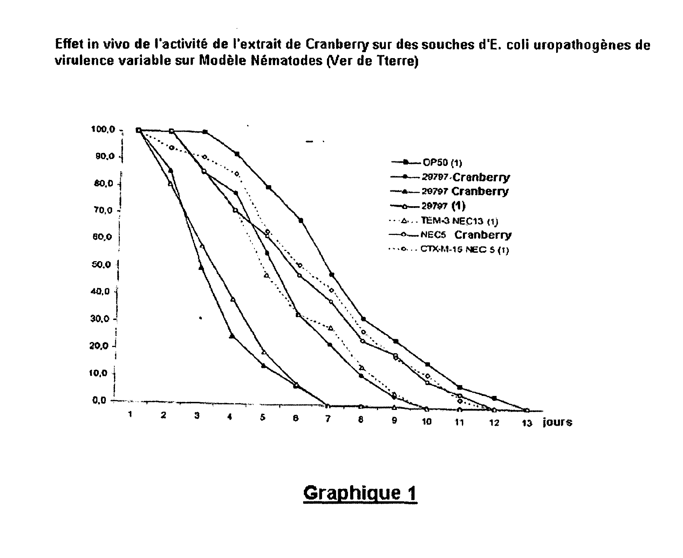

## Revendications

1. Nouvelles compositions antibactériennes **caractérisées en ce qu'**elles associent un extrait de Vaccinium macrocarpon et un agent antibactérien urinaire actif contre les bactéries à Gram+ et à Gram- dans un excipient ou un véhicule inerte, non toxique, pharmceutiquement acceptable.

2. Compositions antibactériennes selon la revendication 1 dans lesquelles l'excipient ou le véhicule sont un de ceux destinés à la voie orale, topique ou parentérale.

3. Compositions antibactériennes selon la revendication 1 dans lesquelles l'extrait de Vaccinium macrocarpon possède une teneur en proanthocyanidines d'au moins 1,35 %.

4. Compositions antibactériennes selon la revendication 1 dans lesquelles l'extrait de Vaccinium macrocarpon possède une teneur en proanthocyanidines d'au moins 20 %.

5. Compositions antibactériennes selon la revendication 1 dans lesquelles l'extrait de Vaccinium marcrocarpon possède une teneur en proanthocyanidines variant de 25 à 50%.

6. Compositions antibactériennes selon la revendication 1 dans lesquelles l'extrait de Vaccinium marcrocarpon est un extrait concentré absorbé sur un matériau poreux inerte ou sur une substance inerte.

7. Compositions antibactériennes selon la revendication 1 dans lesquelles l'agent anti-bactérien est un agent antibactérien éliminé par voie rénale.

8. Compositions antibactériennes selon la revendication 1 dans lesquelles l'agent anti-bactérien est un dérivé du furfural.

9. Compositions antibactériennes selon la revendication 1 dans lesquelles l'agent anti-bactérien est un dérivé de sulfamide.

10. Compositions antibactériennes selon la revendication 1 dans lesquelles l'agent anti-bactérien est un antibiotique du groupe des aminosides.

11. Compositions antibactériennes selon la revendication 1 dans lesquelles l'agent anti-bactérien est la fosfomycine ou un de ses sels.

12. Compositions antibactériennes selon la revendication 1 dans lesquelles l'agent anti-bactérien est un dérivé de tétracycline.

13. Compositions antibactériennes selon la revendication 1 dans lesquelles l'agent anti-bactérien est un de ceux appartenant à la famille des quinolones fluorées ou non fluorées.

14. Compositions antibactériennes selon la revendication 1 dans lesquelles l'agent anti-bactérien est un antibiotique de la famille des beta-lactamines.

15. Compositions antibactériennes selon la revendication 1 dans lesquelles les formes solides contiennent de 0,100 à 0,500 g d'extrait de jus de Cranberry par prise unitaire.

16. Compositions antibactériennes selon la revendication 1 qui contiennent de 0g100 à 0g500 d'agent antibactérien par prise unitaire.

17. Compositions antibactériennes selon la revendication 1 qui contiennent de 0,150 à 0,400 g d'extrait de Cranberry et de 0g100 à 0g200 de Norfloxacine ou de Ciprofloxacine par prise unitaire.

## Revendications modifiées

### Revendications modifiées conformément à la règle 137(2) CBE.

**1.** Nouvelles compositions antibactériennes **caractérisées en ce qu'**elles associent un extrait de Vaccinium macrocarpon et un agent antibactérien actif contre les bactéries à Gram+ et à Gram- choisies dans le groupe constitué par E. coli, Proteus, Klebsiella et Enterobacter dans un excipient ou un véhicule inerte, non toxique, pharmaceutiquement acceptable.

**2.** Compositions antibactériennes selon la revendication 1 dans lesquelles l'excipient ou le véhicule sont un de ceux destinés à la voie orale, topique ou parentérale.

**3.** Compositions antibactériennes selon la revendication 1 dans lesquelles l'extrait de Vaccinium macrocarpon possède une teneur en proanthocyanidines d'au moins 1,35 % m/m.

**4.** Compositions antibactériennes selon la revendication 1 dans lesquelles l'extrait de Vaccinium macrocarpon possède une teneur en proanthocyanidines d'au moins 20 % m/m.

**5.** Compositions antibactériennes selon la revendication 1 dans lesquelles l'extrait de Vaccinium macrocarpon possède une teneur en proanthocyanidines variant de 25 à 50 % m/m.

**6.** Compositions antibactériennes selon la revendication 1 dans lesquelles l'extrait de Vaccinium marcrocarpon est un extrait concentré, absorbé sur un matériau poreux inerte ou sur une substance inerte.

**7.** Compositions antibactériennes selon la revendication 1 dans lesquelles l'agent antibactérien est un dérivé du furfural, choisi parmi la furadoïne et la nitrofurantoïne.

**8.** Compositions antibactériennes selon la revendication 1 dans lesquelles l'agent antibactérien est un dérivé de sulfamide choisi parmi la sulfadiazine et la sulfamerazine.

**9.** Compositions antibactériennes selon la revendication 1 dans lesquelles l'agent antibactérien est un antibiotique du groupe des aminosides, choisi parmi la pentamycine, la dibekacine et l'amikacine.

**10.** Compositions antibactériennes selon la revendication 1 dans lesquelles l'agent antibactérien est la fosfomycine ou un de ses sels.

**11.** Compositions antibactériennes selon la revendication 1 dans lesquelles l'agent antibactérien est un dérivé de tétracycline choisi parmi la doxycycline, la minocycline et l'aureomycine.

**12.** Compositions antibactériennes selon la revendication 1 dans lesquelles l'agent antibactérien est un de ceux appartenant à la famille des quinolones fluorées ou non fluorées, choisies parmies l'acide nalidixique, l'acide oxolinique, la cinnoxaxine, la ciprofloxacine, la norfloxacine, l'ofloxacine, la levofloxacine, la mifloxacine, la lomefloxacine et la sparfloxacine.

**13.** Compositions antibactériennes selon la revendication 1 dans lesquelles l'agent antibactérien est un antibiotique de la famille des beta-lactamines choisi parmi les pénicillines semi-synthétiques ou les cephalosporines choisies parmi la cefoperazone, la ceftazidine et le cefuroxime.

**14.** Compositions antibactériennes selon la revendication 1 dans lesquelles l'agent antibactérien est un nitroimidazole comme le metronidazole.

**15.** Compositions antibactériennes selon la revendication 1 dans lesquelles les formes solides contiennent de 0,100 à 0,500 g d'extrait de jus de Cranberry par prise unitaire.

**16.** Compositions antibactériennes selon la revendication 1 qui contiennent de 0g100 à 0g500 d'agent antibactérien par prise unitaire.

**17.** Compositions antibactériennes selon la revendication 1 qui contiennent de 0,150 à 0,400 g d'extrait de Cranberry et de 0g100 à 0g200 de Norfloxacine ou de Ciprofloxacine par prise unitaire.
